# FASCICULE DE BREVET EUROPEEN

(11) **EP 3 534 970 B1**
(45) Date de publication et mention de la délivrance du brevet: **01.01.2025**
(21) Numéro de dépôt: 17803823.8
(22) Date de dépôt: 07.11.2017
(51) Int. Cl.: A61L 2/10, B29C 49/06, B67B 3/00

(54) **DISPOSITIF ET PROCÉDÉ DE DÉCONTAMINATION**
DEKONTAMINATIONSVORRICHTUNG UND VERFAHREN
DECONTAMINATION DEVICE AND METHOD

(30) Priorité: 07.11.2016 FR 1660741
(43) Date de publication de la demande: 11.09.2019
(73) Titulaire: Claranor, 84140 Montfavet (FR)
(72) Inventeur: FRANC, Janyce, 84000 Avignon (FR); RIEDEL, Christophe, 84000 Avignon (FR)
(74) Mandataire: IPAZ
(86) Numéro de dépôt international: PCT/EP2017/078478
(87) Numéro de publication internationale: WO 2018/083340

(56) Documents cités:
- EP-A1- 1 918 208
- EP-A1- 2 805 912
- EP-A2- 2 138 298
- EP-B1- 2 094 312
- EP-B1- 2 961 437
- WO-A1-2013/092879
- JP-A- H11 263 322
- US-A- 6 037 598
- US-A1- 2012 273 693

## Description

### Domaine technique

La présente invention concerne un dispositif et un procédé de décontamination.

Un tel dispositif permet à un utilisateur de décontaminer un objet comme par exemple une préforme plastique sur une ligne de fabrication et remplissage d'une bouteille ou récipient plastique. Le domaine de l'invention est plus particulièrement celui de la décontamination microbiologique.

### Etat de la technique antérieure

On connaît des procédés de décontamination de préforme.

WO 2013/092879 décrit un ensemble constitué d'un dispositif de décontamination et d'au moins une préforme, une installation et un procédé de fabrication d'un récipient stérile.

EP2094312 décrit un four et une installation pour la fabrication de récipients stériles à partir de préformes en matière thermoplastique décontaminées.

Le document US2014/0265039 décrit un procédé dans lequel des moyens d'émission d'un rayonnement ultra-violet sont introduits à l'intérieur de la préforme pour la décontaminer. On connaît aussi les documents EP 2 138 298 A2, EP 2 961 437 B1, US 2012/273693 A1, JP H11 263322 A and EP 1 918 208 A1.

L'utilisation d'ultra-violet à l'intérieur de la préforme, c'est-à-dire au plus proche de la surface la plus importante à décontaminer, permet un bon niveau de décontamination mais a pour inconvénients :
- de ralentir la cadence de décontamination, et/ou
- d'avoir un dispositif très volumineux comprenant de nombreux postes à lampes UV afin de garantir les cadences de la ligne, et/ou
- de risquer de briser des éléments à l'intérieur de la préforme et d'y laisser des débris, et/ou
- de ne pas décontaminer la partie extérieure du col de la préforme.

Le but de la présente invention est de résoudre au moins un de ces inconvénients tout en continuant de fournir un bon niveau de décontamination de la surface interne de la préforme compatible par exemple avec l'industrie agro-alimentaire, en particulier avec les lignes « ultra-propres » ayant un niveau d'asepsie intermédiaire.

### Exposé de l'invention

Cet objectif est atteint avec un procédé de décontamination d'un tube dont une première extrémité ouverte forme un col intérieur vers l'intérieur du tube et un col extérieur vers l'extérieur du tube, tel que défini par les revendications. Notamment, ledit procédé comprend :
- une élévation initiale de température du tube, puis
- une soumission du tube à un rayonnement ultra-violet, comprenant une émission du rayonnement ultra-violet par des moyens d'émission de rayonnement, et une soumission de l'intérieur du tube, du col extérieur et du col intérieur au rayonnement ultra-violet, de préférence alors que la première extrémité est introduite à l'intérieur d'une cavité agencée pour réfléchir le rayonnement ultra-violet émis par les moyens d'émission et pour diriger ce rayonnement ultraviolet à l'intérieur du tube par la première extrémité, sur le col extérieur du tube et sur le col intérieur du tube.

Le tube comprend :
- une deuxième extrémité formant un fond du tube, et
- un corps s'étendant entre la première extrémité et la deuxième extrémité.

Lors de la soumission du tube au rayonnement ultra-violet, la deuxième extrémité du tube ne se trouve pas dans la cavité.

Lors de la soumission du tube au rayonnement ultra-violet, le corps du tube peut ne pas se trouver dans la cavité.

Toutes les parties du tube (ou toutes les parties du tube sauf le col) ont de préférence une température supérieure ou égale à 85°C lors de la soumission du tube au rayonnement ultra-violet.

Lors de la soumission du tube au rayonnement ultra-violet, les moyens d'émission de rayonnement peuvent ne pas être insérés à l'intérieur du tube.

Le procédé selon l'invention ne comprend de préférence aucune insertion d'aucun autre moyen d'émission de rayonnement à l'intérieur du tube.

Le rayonnement ultra-violet est de préférence de la lumière ultra-violette pulsée ayant :
- une durée de pulse inférieur à 500 µs, et/ou
- une énergie lumineuse par pulse d'au moins 70 Joules, et/ou
- une fréquence de pulses d'au moins 0,5 Hz, ou bien plus généralement une fréquence de pulses contrôlée en fonction de la vitesse de défilement des tubes à décontaminer.

Le tube peut être un tube en matière plastique.

Le procédé selon l'invention peut comprendre au moins une des étapes suivantes après la soumission du tube au rayonnement ultra-violet, de préférence dans l'ordre suivant:
- une modification de la forme tube, et/ou
- un remplissage du tube, et/ou
- une fixation d'un bouchon sur le tube en contact avec le col extérieur.

Le procédé selon l'invention peut ne pas comprendre d'élévation de la température du tube :
- entre la soumission du tube au rayonnement ultra-violet et la modification de la forme du tube, et/ou
- entre la soumission du tube au rayonnement ultra-violet et le remplissage du tube, et/ou
- entre la soumission du tube au rayonnement ultra-violet et la fixation du bouchon.

Le procédé selon l'invention peut comprendre une soumission du bouchon à un rayonnement ultra-violet avant sa fixation sur le tube.

Le procédé selon l'invention peut comprendre aucune décontamination chimique du tube.

L'élévation initiale de température peut être obtenue par passage du tube dans un four et/ou par soumission du tube à une lampe à infra-rouge.

L'élévation initiale de température élève de préférence toutes les parties du tube à une température supérieure ou égale à 90°C.

Suivant encore un autre aspect de l'invention, il est proposé un dispositif de décontamination d'un tube, tel que défini dans la revendication 14.

Les moyens de déplacement et la cavité peuvent être agencés ensemble pour que le corps du tube ne se trouve pas dans la cavité lors du passage du tube au niveau de la cavité.

Les moyens de chauffage, la cavité et les moyens de déplacement reliant les moyens de chauffage à la cavité peuvent être agencés pour que toutes les parties du tube aient une température supérieure ou égale à 85°C lors du passage du tube au niveau de la cavité.

Les moyens d'émission de rayonnement peuvent ne pas être agencés pour être insérés à l'intérieur du tube lors du passage du tube au niveau de la cavité.

Le dispositif selon l'invention peut ne comprendre aucun autre moyen d'émission de rayonnement agencé pour être inséré à l'intérieur du tube lors du passage du tube au niveau de la cavité.

Les moyens d'émission peuvent être agencés pour émettre le rayonnement ultra-violet sous la forme de lumière ultra-violette pulsée ayant :
- une durée de pulse inférieur à 500 µs, et/ou
- une énergie lumineuse par pulse d'au moins 70 Joules, et/ou
- une fréquence de pulses d'au moins 0,5 Hz, ou bien plus généralement une fréquence de pulses contrôlée en fonction de la vitesse de défilement des tubes à décontaminer.
En outre, l'invention concerne également un dispositif tel que défini par les revendications.

Le dispositif selon l'invention peut comprendre au moins un des moyens suivants situé(s) sur le chemin de décontamination en aval de la cavité par rapport au sens de parcours, de préférence dans l'ordre suivant par rapport au sens de parcours :
- des moyens pour modifier la forme du tube, et/ou
- des moyens pour remplir le tube, et/ou
- des moyens de fixation agencés pour fixer un bouchon sur le tube en contact avec le col extérieur du tube.

Le dispositif selon l'invention peut ne pas comprendre de moyens pour élever la température du tube :
- le long du chemin de décontamination entre la cavité et les moyens pour modifier la forme du tube, et/ou
- le long du chemin de décontamination entre la cavité et les moyens pour remplir le tube, et/ou
- le long du chemin de décontamination entre la cavité et les moyens pour fixer le bouchon.

Le dispositif selon l'invention peut comprendre des moyens pour soumettre le bouchon à un rayonnement ultra-violet avant sa fixation sur le tube par les moyens de fixation.

Le dispositif selon l'invention peut comprendre aucun moyen de décontamination chimique du tube.

Les moyens de chauffage peuvent comprendre un four et/ou une lampe à infra-rouge.

Les moyens de chauffage peuvent être agencés pour élever toutes les parties du tube à une température supérieure ou égale à 90°C.

### Description des figures et modes de réalisation

D'autres avantages et particularités de l'invention apparaîtront à la lecture de la description détaillée de mises en œuvre et de modes de réalisation nullement limitatifs, et des dessins annexés suivants :
- la figure 1 est une vue schématique d'un mode de réalisation de dispositif 1 selon l'invention mettant en œuvre un mode de réalisation de procédé selon l'invention, qui sont des modes de réalisation préférés de l'invention,
- la figure 2 est une vue schématique de profil de la position d'un tube 2 inséré à l'intérieur d'une cavité 4 du dispositif 1 de la figure 1, et
- la figure 3 illustre un profil d'intensité de chaque lampe UV présente dans la cavité 4 en fonction de la longueur d'onde λ (en nm) émise par cette lampe UV.

Ces modes de réalisation étant nullement limitatifs, on pourra notamment considérer des variantes de l'invention ne comprenant qu'une sélection de caractéristiques décrites ou illustrées par la suite isolées des autres caractéristiques décrites ou illustrées (même si cette sélection est isolée au sein d'une phrase comprenant ces autres caractéristiques), si cette sélection de caractéristiques est suffisante pour conférer un avantage technique ou pour différencier l'invention par rapport à l'état de la technique antérieure. Cette sélection comprend au moins une caractéristique de préférence fonctionnelle sans détails structurels, et/ou avec seulement une partie des détails structurels si cette partie uniquement est suffisante pour conférer un avantage technique ou à différencier l'invention par rapport à l'état de la technique antérieure.

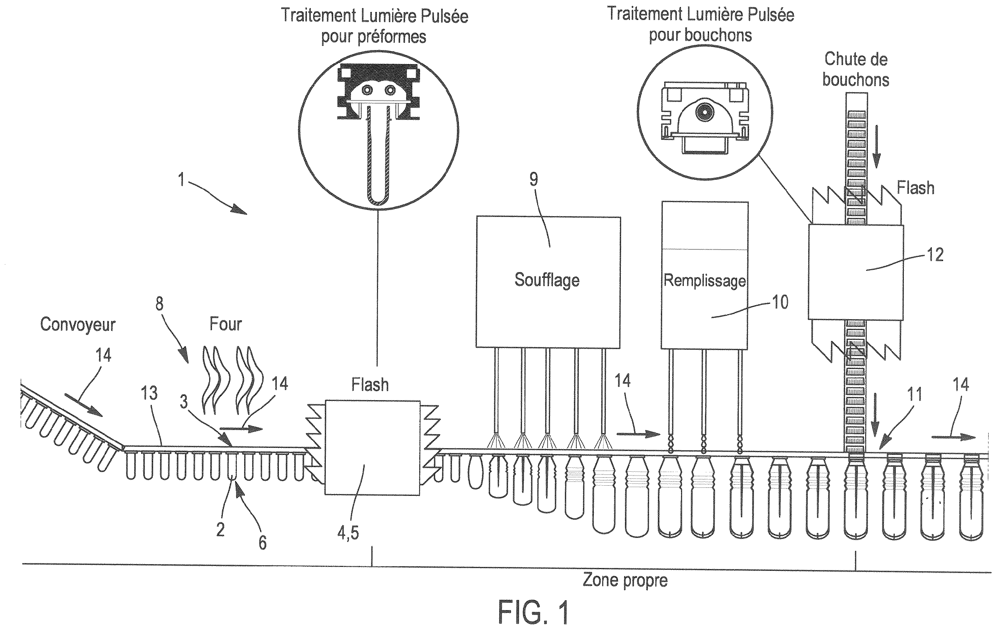

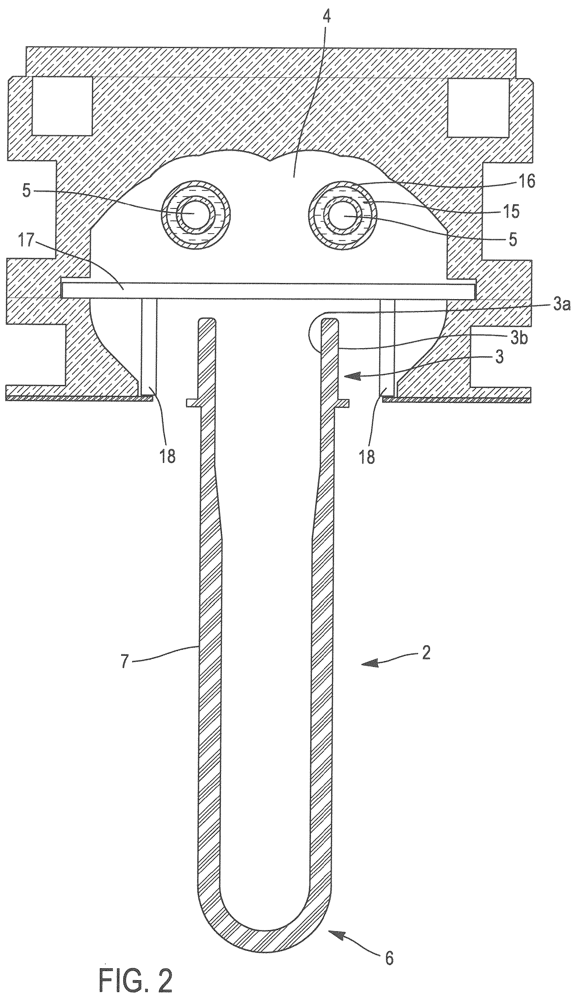

On va tout d'abord décrire, en référence aux figures 1 à 3, un mode de réalisation de dispositif 1 selon l'invention mettant en œuvre un mode de réalisation de procédé selon l'invention.

Le dispositif 1 de décontamination microbiologique d'un tube 2 comprend des moyens 13 pour déplacer, le long d'un chemin de décontamination selon un sens de parcours 14, un tube 2.

Ces moyens de déplacement comprennent par exemple un convoyeur portant le tube 2, par exemple :
- du type à rail mobile en inox, et/ou
- du type à « pince ». Dans ce cas, le transport des préformes entre le four et la souffleuse a typiquement lieu par des pinces qui attrapent les préformes sous la collerette. En effet, dans le four, un mandrin vient tenir la préforme par l'intérieur du goulot, et il fait tourner la préforme sur elle-même en même temps qu'elle se déplace en face des rampes infra-rouges. La préforme ne peut être tenue par son intérieur. Avant que le mandrin ne lâche la préforme, elle est prise par une pince, qui est montée sur un carrousel, et qui attrape la préforme, l'écarte de la précédente (dans le four les préformes sont espacées de 35mm, dans la souffleuse d'environ 400mm du fait de l'encombrement des moules), par accélération, avant que la préforme ne soit reprise par le moule qui lui aussi est monté sur un carrousel.

Le tube 2 comprend :
- une première extrémité ouverte 3 qui forme un col intérieur 3a vers l'intérieur du tube 2 et un col extérieur 3b vers l'extérieur du tube 2,
- une deuxième extrémité fermée 6 formant un fond du tube 2, et
- un corps 7 s'étendant entre la première extrémité 3 et la deuxième extrémité 6.

Le dispositif 1 comprend des moyens de chauffage initial 8, agencés pour élever la température du tube 2 sur le chemin de décontamination.

Les moyens de chauffage 8 comprennent un four, plus exactement un four à lampe halogène à infra-rouge de 1000 Watts ou même de plusieurs dizaines de kilo Watts pour un plus grand débit.

Les moyens de chauffage 8 sont agencés pour élever toutes les parties du tube 2 à une température supérieure ou égale à 90°C, de préférence supérieure ou égale à 100°C, de préférence supérieure ou égale à 115°C, de préférence supérieure ou égale à 142°C.

Le dispositif 1 comprend des moyens pour soumettre le tube 2 à un rayonnement ultra-violet, plus exactement pour soumettre :
- le col intérieur 3a du tube 2,
- le col extérieur 3b du tube 2, et
- l'intérieur du tube 2, plus exactement :
   ∘ le col intérieur 3a déjà mentionné
   ∘ le corps 7 du tube du côté de l'intérieur du tube 2
   ∘ le fond 6 du tube 2 du côté de l'intérieur du tube 2
   à un rayonnement ultra-violet.

Ces moyens pour soumettre le tube 2 à un rayonnement ultra-violet comprennent des moyens d'émission 5 agencés pour émettre le rayonnement ultra-violet.

Le rayonnement ultra-violet a au moins une longueur d'onde comprise entre 10 nm et 380 nm, de préférence entre 180 nm et 380 nm.

Les moyens d'émission 5 sont en outre agencés pour émettre, simultanément au rayonnement ultra-violet, au moins une longueur d'onde comprise entre 380 et 1100 nm, ce qui est avantageux par exemple contre les moisissures.

Ces moyens d'émission 5 comprennent typiquement au moins une lampe UV.

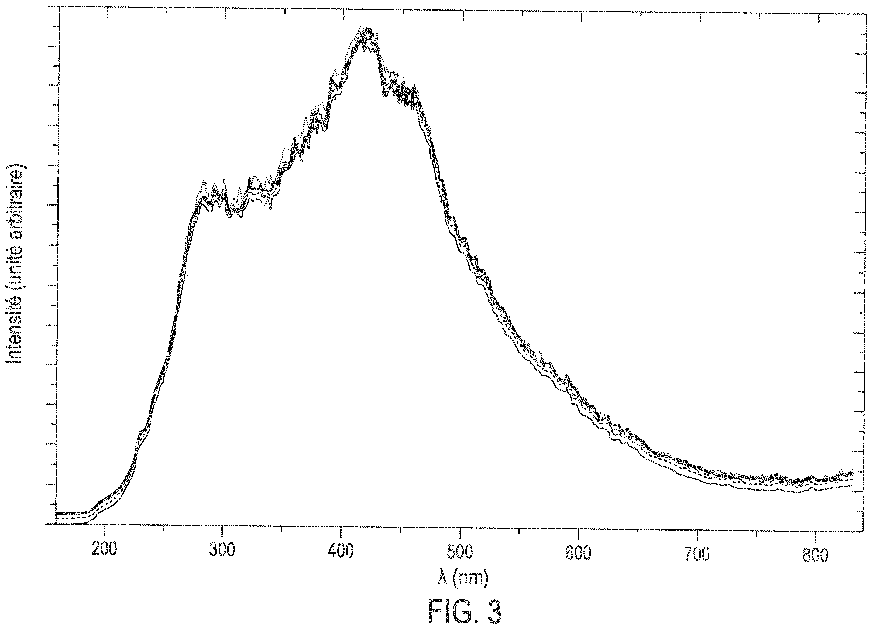

Chaque lampe UV s'étend selon le sens de parcours 14, et comprend par exemple une lampe UV à ionisation de gaz Xénon dont le profil d'intensité (en unité arbitraire, l'intensité dépendant de la tension d'alimentation) est illustré sur la figure 3. Pour chaque lampe, une tension d'alimentation de 2100V correspond à une énergie totale dissipée (lumière, chaleur, etc.) par pulse de 145 Joules soit environ 72,5 joules d'énergie lumineuse émise par la lampe ; Pour chaque lampe, une tension d'alimentation de 2500V correspond à une énergie totale dissipée (lumière, chaleur, etc.) par pulse de 206 Joules soit environ 103 Joules d'énergie lumineuse émise par la lampe.

Chaque lampe UV est en contact d'un liquide de refroidissement 15 (typiquement de l'eau désionisée maintenue typiquement entre 10°C et 35°C) situé entre la lampe UV est une fenêtre 16 en quartz.

Les moyens pour soumettre le tube 2 à un rayonnement ultra-violet comprennent en outre une cavité 4 située sur le chemin de décontamination en aval des moyens de chauffage 8 par rapport au sens de parcours 14.

Les moyens de déplacement 13 et la cavité 4 sont agencés ensemble l'un par rapport à l'autre pour que :
- la première extrémité 3 du tube 2 soit introduite à l'intérieur de la cavité 4 lors d'un passage du tube 2 au niveau de la cavité 4,
- la deuxième extrémité 6 du tube 2 ne se trouve pas dans la cavité 4 lors du passage du tube 2 au niveau de la cavité 4,
- le corps 7 du tube 2 ne se trouve pas dans la cavité 4 lors du passage du tube 2 au niveau de la cavité 4.

La cavité 4 est agencée pour réfléchir le rayonnement ultra-violet émis par les moyens d'émission 5 et pour diriger ce rayonnement ultraviolet :
- sur le col intérieur 3a du tube 2
- sur le col extérieur 3b du tube 2
- à l'intérieur du tube 2 par la première extrémité 3, plus exactement :
   ∘ sur le col intérieur 3a déjà mentionné
   ∘ sur le corps 7 du tube 2 du côté de l'intérieur du tube 2
   ∘ sur le fond 6 du tube 2 du côté de l'intérieur du tube 2
de manière à soumettre tout l'intérieur du tube 2, tout le col extérieur 3b et tout le col intérieur 3a au rayonnement ultra-violet lors du passage du tube 2 au niveau de la cavité 4.

Les moyens d'émission 5 sont disposés à l'intérieur de la cavité 4.

L'intérieur de la cavité 4 est délimitée par une surface réfléchissante
en Aluminium, réalisée dans une pièce en EN AW 5754.

Les moyens de chauffage 8, la cavité 4 et les moyens de déplacement 13 reliant les moyens de chauffage 8 à la cavité 4 sont agencés pour que toutes les parties du tube 2 aient une température :
- supérieure ou égale à une température de transformation de la forme du tube 2 ;
- de préférence supérieure ou égale à 85°C, de préférence supérieure ou égale à 95°C, de préférence supérieure ou égale à 110°C, de préférence supérieure ou égale à 137°C (par exemple dans le cas du présent mode de réalisation pour lequel la chute de température du tube 2 entre les moyens 8 et la cavité 4 est d'au plus 5 °C), ou
- de préférence supérieure ou égale à 90°C, de préférence supérieure ou égale à 100°C, de préférence supérieure ou égale à 115°C, de préférence supérieure ou égale à 142°C (par exemple dans un cas idéal pour lequel il n'y a pas de chute de température du tube 2 (arrondie à 1°C près) entre les moyens 8 et la cavité 4 est d'au plus 5 °C)
lors du passage du tube 2 au niveau de la cavité 4.

Pour cela :
- la distance entre la sortie des moyens de chauffage 8 et l'entrée de la cavité 4 est par exemple au maximum de 100 cm, typiquement de 30cm ou 50 cm et/ou
- les moyens de déplacement 13 sont par exemple agencés pour déplacer le tube 2 à une vitesse d'au moins 0,1 m.s⁻¹, de préférence d'au moins 1m.s⁻¹ entre la sortie des moyens de chauffage 8 et l'entrée de la cavité 4. Les moyens de déplacement 13 sont réglables en vitesse, et peuvent aussi fonctionner à une vitesse plus lente.

On remarque que les moyens d'émission de rayonnement 5 ne sont pas agencés pour être insérés à l'intérieur du tube 2 lors du passage du tube 2 au niveau de la cavité 4. Ceci permet de ne pas perdre de temps à insérer et sortir les moyens 5 à l'intérieur du tube 2 et permet une grande rapidité du procédé selon l'invention, typiquement d'au moins 25 000 tubes décontaminés par heure (typiquement entre 5000 et 100 000 tubes décontaminés par heure). Ceci permet en outre d'éviter de briser les moyens 5 à l'intérieur du tube 2 et d'y laisser des débris dangereux notamment dans le cadre de l'industrie agro-alimentaire.

De manière générale, le dispositif 1 ne comprend aucun autre moyen d'émission de rayonnement agencé pour être inséré à l'intérieur du tube 2 lors du passage du tube 2 au niveau de la cavité 4

Les moyens d'émission 5 sont agencés pour émettre le rayonnement ultra-violet sous la forme de lumière ultra-violette pulsée ayant :
- au moins une longueur d'onde UV comprise entre 180 nm et 380 nm, et
- une durée de pulse inférieur à 500 µs, typiquement supérieure ou égale à 250 µs et/ou inférieure ou égale à 300µs et/ou
- une énergie lumineuse par pulse, entre 180 nm et 1100 nm, d'au moins 70 Joules, de préférence d'au moins 100 Joules, de préférence d'au moins 150 Joules (par pulse issu de la combinaison des lampes UV qui émettent simultanément), dont au moins 5% (de préférence au moins 10%) entre 180 nm et 380 nm et/ou dont au moins 50% (de préférence au moins 60%) entre 400 nm et 700 nm, et/ou
- une fréquence de pulses d'au moins 0,1 Hz, typiquement supérieure à 0,5 Hz et/ou inférieure à 20 Hz, ou bien plus généralement une production de pulses contrôlée en fonction de la vitesse de défilement des tubes à décontaminer devant les moyens d'émission 5 et/ou dans la cavité 4.

Comme illustré sur la figure 3, le rayonnement ultra-violet est un flash de lumière (typiquement un flash de lumière blanche) comprenant des UV et comprenant en outre au moins une autre longueur d'onde comprise entre 400 et 700 nm, c'est-à-dire de la lumière « visible ».

Ce rayonnement combine trois points :
- l'émission d'UV
- la forte puissance, autrement dit la brièveté de l'émission (qui donne l'effet puissance)
- le spectre « continu », i.e. la multiplicité des longueurs d'ondes.

Le dispositif 1 comprend en outre, sur le chemin de décontamination en aval de la cavité 4 par rapport au sens de parcours 14, et dans l'ordre suivant par rapport au sens de parcours 14:
- des moyens 9 pour modifier la forme du tube 2, de préférence par soufflage, le tube étant une préforme encore réchauffée par les moyens de chauffage 8 à une température suffisamment élevée pour sa déformation ; on donne par exemple au tube 2 une forme de bouteille ; Les moyens 9 peuvent par exemple consister en une souffleuse.
- des moyens 10 pour remplir le tube 2 par un liquide ou produit alimentaire ou autre du côté de sa première extrémité 3 ; Les moyens 10 peuvent par exemple consister en une remplisseuse de matière pâteuse, gel, liquide naturel, gazeux ou de type produit laitier ou autre.
- des moyens de fixation 11 agencés pour fixer un bouchon sur le tube 2 en contact avec le col extérieur 3b du tube 2; Les moyens 11 peuvent par exemple consister en une boucheuse, une visseuse, etc.

Les moyens de chauffage 8 sont utilisés à la fois pour la décontamination du tube 2 et pour rendre possible la modification de la forme du tube 2.

Ceci est rendu possible par le passage très rapide du tube 2 dans la cavité 4, sans perdre de temps à insérer et ressortir du tube 2 les moyens d'émission 5, ce qui évite un refroidissement trop important du tube 2.

Le dispositif 1 ne comprend pas de moyens pour élever la température du tube 2 le long du chemin de décontamination entre la cavité 4 et les moyens 9 pour modifier la forme du tube 2, plus généralement entre la cavité 4 et les moyens 10 pour remplir le tube 2, plus généralement entre la cavité 4 et les moyens 11 pour fixer le bouchon.

Le dispositif 1 comprend des moyens 12 pour soumettre le bouchon à un rayonnement ultra-violet avant sa fixation sur le tube 2 par les moyens de fixation 11, du même type que ceux décrits pour les moyens 5 et la cavité 4.

On remarque enfin que le dispositif 1 ne comprend aucun moyen de décontamination chimique du tube. En particulier, entre les moyens de chauffage 8 et les moyens de fixation11, le dispositif 1 ne comprend aucun moyen pour mettre le tube 2 en contact d'un autre liquide ou produit que celui utilisé par les moyens de remplissage 10 pour remplir le tube et conservé à l'intérieur du tube 2 après la fixation du bouchon.

Pour des raisons de sécurité, les lampes UV sont séparées du tube 2 par des lames de quartz 17, 18 disposées au sein de la cavité 4.

Le mode de réalisation de procédé de décontamination microbiologique du tube 2 mis en œuvre par le dispositif 1 comprend :
- une élévation initiale de température du tube 2 (c'est-à-dire d'au moins une partie du tube ; de préférence de toutes les parties du tube ou de toutes les parties du tube sauf le col) par les moyens de chauffage 8, obtenue par passage du tube 2 dans le four à lampe à infra-rouge ; l'élévation initiale de température élève toutes les parties du tube à une température supérieure ou égale à 90°C, de préférence supérieure ou égale à 100°C, de préférence supérieure ou égale à 115°C, de préférence supérieure ou égale à 142°C ; puis
- une soumission du tube 2 au rayonnement ultra-violet, comprenant une émission du rayonnement ultra-violet par les moyens d'émission 5, et une soumission de l'intérieur du tube (plus exactement du col intérieur 3a, du corps 7 du tube 2 du côté de l'intérieur du tube 2 et du fond 6 du tube 2 du côté de l'intérieur du tube 2, de préférence de tout le col intérieur 3a, de tout le corps 7 du tube 2 du côté de l'intérieur du tube 2 et de tout le fond 6 du tube 2 du côté de l'intérieur du tube 2), du col extérieur 3b (de préférence de tout le col extérieur 3b) et du col intérieur 3a (de préférence de tout le col intérieur 3a) au rayonnement ultra-violet, alors que la première extrémité 3 est introduite à l'intérieur de la cavité 4.

Lors de la soumission du tube 2 au rayonnement ultra-violet, la deuxième extrémité 6 du tube ne se trouve pas dans la cavité 4.

Lors de la soumission du tube 2 au rayonnement ultra-violet, le corps 7 du tube ne se trouve pas dans la cavité 4.

Toutes les parties du tube 2 ont une température :
- supérieure ou égale à une température de transformation de la forme du tube 2 ;
- de préférence supérieure ou égale à 85°C, de préférence supérieure ou égale à 95°C, de préférence supérieure ou égale à 110°C, de préférence supérieure ou égale à 137°C (par exemple dans le cas du présent mode de réalisation pour lequel la chute de température du tube 2 entre les moyens 8 et la cavité 4 est d'au plus 5 °C), ou
- de préférence supérieure ou égale à 90°C, de préférence supérieure ou égale à 100°C, de préférence supérieure ou égale à 115°C, de préférence supérieure ou égale à 142°C (par exemple dans un cas idéal pour lequel il n'y a pas de chute de température du tube 2 (arrondie à 1°C près) entre les moyens 8 et la cavité 4 est d'au plus 5 °C)
lors de la soumission du tube 2 au rayonnement ultra-violet.

Pour cela :
- la distance entre la sortie des moyens de chauffage 8 et l'entrée de la cavité 4 est par exemple au maximum de 100 cm, et/ou
- les moyens de déplacement 13 déplacent le tube 2 à une vitesse d'au moins 0,1 m.s⁻¹ , de préférence d'au moins 1m.s⁻¹ entre la sortie des moyens de chauffage 8 et l'entrée de la cavité 4. Les préformes sont de préférence accélérées : pour 72000/h (20/s) elles passent de 0,7m/s à 7m/s.

Lors de la soumission du tube 2 au rayonnement ultra-violet, les moyens d'émission 5 de rayonnement ne sont pas insérés à l'intérieur du tube 2. De manière générale, il n'y a aucune insertion d'aucun autre moyen d'émission de rayonnement à l'intérieur du tube 2.

Le rayonnement ultra-violet est de la lumière ultra-violette pulsée telle que précédemment décrite.

Le tube 2 est un tube en matière plastique, par exemple :
- en polytéréphtalate d'éthylène (PET)
- de 22,5 grammes
- avec un diamètre intérieur du tube de 28 mm du côté de l'extrémité 3
- d'une hauteur de 90 mm.

Cependant, on remarque que le principe de décontamination selon ce mode de réalisation est compatible avec la variante dans laquelle le tube 2 peut être de tous types (verre, acide polylactique (PLA), etc.)

Le mode de réalisation de procédé selon l'invention mis en œuvre dans le dispositif 1 comprend les étapes suivantes après la soumission du tube 2 au rayonnement ultra-violet, dans l'ordre suivant:
- une modification de la forme tube 2 par soufflage par les moyens 9,
- un remplissage du tube 2 avec un liquide ou produit alimentaire ou autre par les moyens 10, et
- une fixation d'un bouchon sur le tube 2 en contact avec le col extérieur 3b, par les moyens 11, alors que le liquide ou produit est toujours contenu dans le tube 2, mais après avoir soumis le bouchon à un rayonnement ultra-violet par les moyens 12.

On remarque que ce mode de réalisation de l'invention permet d'être très rapide tout en :
- limitant les risques de débris dans le tube 2,
- permettant un dispositif 1 très compact,
- étant relativement économique et facile d'entretien,
- se passant d'une deuxième élévation de la température du tube 2, en particulier :
   ∘ entre la soumission du tube 2 au rayonnement ultra-violet et la modification de la forme du tube 2, et/ou
   ∘ entre la soumission du tube 2 au rayonnement ultra-violet et le remplissage du tube 2 (dans le cas où la déformation par soufflage ne souffle pas d'air chaud à une température supérieure à celle du tube 2), et/ou
   ∘ entre la soumission du tube 2 au rayonnement ultra-violet et la fixation du bouchon 2 (dans le cas où la déformation par soufflage ne souffle pas d'air chaud à une température supérieure à celle du tube 2).
- se passant de décontamination chimique potentiellement problématique dans l'industrie agroalimentaire; en effet ce mode de réalisation de procédé selon l'invention ne comprend aucune décontamination chimique du tube 2. En particulier, entre l'élévation initiale de température (par les moyens 8) et la fixation du bouchon (par les moyens 11), le tube 2 n'est en contact d'aucun autre liquide ou produit que celui utilisé dans l'étape de remplissage (par les moyens 10) et conservé à l'intérieur du tube 2 après la fixation du bouchon.
- assurant un niveau de décontamination très performant de l'intérieur du tube 2 (typiquement d'au moins 1 log, de préférence d'au moins 1,5 log, de préférence d'au moins 3 log, typiquement entre 3 et 5 log), destiné à être en contact du produit alimentaire, et du col extérieur 3b, destiné à être en contact avec le bouchon et/ou la bouche d'un utilisateur.

Le niveau de décontamination atteint est même surprenant et inattendu, et on constate un effet de synergie entre le chauffage par les moyens 8 et la décontamination UV par les moyens 5 dont l'effet est supérieur à la somme des effets du chauffage seul et de la décontamination UV seule.

Dans le présent mode de réalisation, la décontamination lumineuse par flash de lumière « blanche » combine deux effets :
- effet photochimique par les longueurs d'onde UV
- effet photothermique par les longueurs d'onde « visibles », certains germes absorbant de la lumière visible et étant ainsi chauffés par le flash de lumière.

Le traitement de décontamination lumineux est immédiat, du fait de l'emploi d'une technologie pulsée, émettant de la lumière blanche riche en UV, avec une très forte puissance.

Des tests ont été réalisés.

Pour chaque tube 2 testé, 5.10⁴ CFU (pour « colony-forming unit » en anglais ou UFC Unité Formant Colonies en Français) de germes Aspergillus brasiliensis DSM 1988 ont été déposés et répartis :
- sur 16 gouttes de 1 µl sur le col intérieur 3a réparties sur 4 lignes verticales espacées d'un angle de 90° par rapport au centre du tube 2, et
- sur 50 gouttes de 1 µl sur le corps 7 et le fond 6 du côté de l'intérieur du tube 2, réparties sur 4 lignes verticales espacées d'un angle de 90° par rapport au centre du tube 2.

On réalise ensuite des mesures de décontamination (chacune sur au moins cinq tubes 2) pour comparer les effets :
- du chauffage seul par les moyens 8
- de la décontamination UV seule par les moyens 4, 5
- de synergie selon l'invention entre le chauffage par les moyens 8 et la décontamination UV de l'intérieur du tube 2 par les moyens 4, 5.

Le tableau ci-dessous donne les valeurs de ces mesures de décontamination pour certains de ces tests.

| **Type de décontamination** | | **Mesure de décontamination (en log)** |
|---|---|---|
| | A) Chauffage seul par les moyens de chauffage 8 à 115 °C (température du tube 2 en sortie du four 8) | 2,1 |
| | B) Chauffage seul par les moyens de chauffage 8 à 142 °C (température du tube 2 en sortie du four 8) | 2,2 |
| | C) décontamination UV seule par les moyens 4, 5 ; 1 flash par lampe UV par tube 2 | 1,9 |
| | D) décontamination UV seule par les moyens 4, 5 ; 2 flashs par lampe UV par tube 2 | 2,0 |
| Cas chauffage A) + décontamination UV D) | | 4,2 |
| Cas chauffage B) + décontamination UV C) | | 4,5 |
| Cas chauffage B) + décontamination UV D) | | 4,4 |

Les décontaminations sont données en log (1 log de décontamination correspond à une division par dix du nombre de germes ; 3 log de décontamination correspond à une division par mille du nombre de germes ; 4 log de décontamination correspond à une division par dix mille du nombre de germes ; etc.). Le nombre de germes restant est dénombré selon les techniques de microbiologie classiques (méthode par inclusion dans un milieu de culture et filtration).

Des tests montrent aussi que l'invention permet d'obtenir sur le col extérieur 3b (surface moins critique) un niveau de décontamination supérieur à 4,2 log.

Bien sûr, l'invention n'est pas limitée aux exemples qui viennent d'être décrits et de nombreux aménagements peuvent être apportés à ces exemples sans sortir du cadre de l'invention.

En particulier, la description du précédent mode de réalisation pour un tube reste valable dans le cadre de l'invention pour plusieurs tubes 2 décontaminés en série les uns à la suite des autres et/ou en parallèle.

Bien entendu, les différentes caractéristiques, formes, variantes et modes de réalisation de l'invention peuvent être associées les unes avec les autres selon diverses combinaisons dans la mesure où elles ne sont pas incompatibles ou exclusives les unes des autres. En particulier toutes les variantes et modes de réalisation décrits précédemment sont combinables entre eux.

Ainsi, dans une variante de procédé selon l'invention, toutes les parties du tube sauf le col ont de préférence une température supérieure ou égale à 85°C lors de la soumission du tube au rayonnement ultra-violet, de préférence supérieure ou égale à 90°C, de préférence supérieure ou égale à 100°C, de préférence supérieure ou égale à 115°C, de préférence supérieure ou égale à 142°C.

De même, dans un dispositif selon l'invention, les moyens de chauffage, la cavité et les moyens de déplacement reliant les moyens de chauffage à la cavité peuvent être agencés pour que toutes les parties du tube sauf le col aient une température supérieure ou égale à 85°C lors du passage du tube au niveau de la cavité, de préférence supérieure ou égale à 90°C, de préférence supérieure ou égale à 100°C, de préférence supérieure ou égale à 115°C, de préférence supérieure ou égale à 142°C.

Le col comprenant typiquement des parties assurant une étanchéité de la future bouteille, cela peut en effet permettre d'éviter de le déformer par la chaleur.

## Revendications

1. Procédé de décontamination d'un tube (2) dont une première extrémité ouverte (3) forme un col intérieur (3a) vers l'intérieur du tube et un col extérieur (3b) vers l'extérieur du tube, ledit procédé comprenant :
- une élévation initiale de température du tube, puis
une soumission du tube à un rayonnement ultra-violet, comprenant une émission du rayonnement ultra-violet par des moyens d'émission (5) de rayonnement, et une soumission de l'intérieur du tube, du col extérieur et du col intérieur au rayonnement ultra-violet, alors que la première extrémité (3) est introduite à l'intérieur d'une cavité (4) agencée pour réfléchir le rayonnement ultra-violet émis par les moyens d'émission et pour diriger ce rayonnement ultraviolet à l'intérieur du tube par la première extrémité, sur le col extérieur du tube et sur le col intérieur du tube (2); ladite tube (2) comprenant :
- une deuxième extrémité (6) formant un fond du tube, et
- un corps (7) s'étendant entre la première extrémité et la deuxième extrémité;
de manière que lors de la soumission du tube au rayonnement ultra-violet, la deuxième extrémité du tube ne se trouve pas dans la cavité.

2. Procédé selon la revendication 1, **caractérisé en ce que**, lors de la soumission du tube au rayonnement ultra-violet, le corps du tube ne se trouve pas dans la cavité.

3. Procédé selon l'une quelconque des revendications précédentes, **caractérisé en ce que** toutes les parties du tube ou toutes les parties du tube sauf le col ont une température supérieure ou égale à 85°C lors de la soumission du tube au rayonnement ultra-violet.

4. Procédé selon l'une quelconque des revendications précédentes, **caractérisé en ce que**, lors de la soumission du tube au rayonnement ultra-violet, les moyens d'émission de rayonnement ne sont pas insérés à l'intérieur du tube.

5. Procédé selon la revendication 4, **caractérisé en ce qu'**il ne comprend aucune insertion d'aucun autre moyen d'émission de rayonnement à l'intérieur du tube.

6. Procédé selon l'une quelconque des revendications précédentes, **caractérisé en ce que** le rayonnement ultra-violet comprend au moins une longueur d'onde UV comprise entre 180 nm et 380 nm et a :
- une durée de pulse inférieur à 500 µs, et/ou
- une énergie lumineuse par pulse d'au moins 70 Joules, et/ou
- une fréquence de pulses d'au moins 0,5 Hz ou une production de pulses contrôlée en fonction de la vitesse de défilement des tubes à décontaminer devant les moyens d'émission (5) et/ou dans la cavité (4).

7. Procédé selon l'une quelconque des revendications précédentes, **caractérisé en ce que** le tube est un tube en matière plastique.

8. Procédé selon l'une quelconque des revendications précédentes, **caractérisé en ce qu'**il comprend au moins une des étapes suivantes après la soumission du tube au rayonnement ultra-violet, de préférence dans l'ordre suivant:
- une modification de la forme tube, et/ou
- un remplissage du tube, et/ou
- une fixation d'un bouchon sur le tube en contact avec le col extérieur.

9. Procédé selon la revendication 8, **caractérisé en ce qu'**il ne comprend pas d'élévation de la température du tube :
- entre la soumission du tube au rayonnement ultra-violet et la modification de la forme du tube, et/ou
- entre la soumission du tube au rayonnement ultra-violet et le remplissage du tube, et/ou
- entre la soumission du tube au rayonnement ultra-violet et la fixation du bouchon.

10. Procédé selon la revendication 8 ou 9,
**caractérisé en ce qu'**il
comprend une soumission du bouchon à un rayonnement ultra-violet avant sa fixation sur le tube.

11. Procédé selon l'une quelconque des revendications précédentes, **caractérisé en ce qu'**il ne comprend aucune décontamination chimique du tube.

12. Procédé selon l'une quelconque des revendications précédentes, **caractérisé en ce que** l'élévation initiale de température est obtenue par passage du tube dans un four et/ou par soumission du tube à une lampe à infra-rouge.

13. Procédé selon l'une quelconque des revendications précédentes, **caractérisé en ce que** l'élévation initiale de température élève toutes les parties du tube à une température supérieure ou égale à 90°C.

14. Dispositif (1) de décontamination d'un tube (2), comprenant :
- des moyens (13) pour déplacer, le long d'un chemin de décontamination selon un sens de parcours (14) , un tube dont une première extrémité ouverte (3) forme un col intérieur (3a) vers l'intérieur du tube et un col extérieur (3b) vers l'extérieur du tube,
- des moyens de chauffage initial (8), agencés pour élever la température du tube sur le chemin de décontamination,
- des moyens pour soumettre le tube à un rayonnement ultra-violet, comprenant des moyens d'émission (5) agencés pour émettre le rayonnement ultra-violet , et une cavité (4) située sur le chemin de décontamination en aval des moyens de chauffage par rapport au sens de parcours, les moyens de déplacement (13) et la cavité (4) étant agencés ensemble pour que la première extrémité (3) soit introduite à l'intérieur de la cavité (4) lors d'un passage du tube au niveau de la cavité (4)
la cavité (4) étant agencée pour réfléchir le rayonnement ultra-violet émis par les moyens d'émission et pour diriger ce rayonnement ultraviolet à l'intérieur du tube par la première extrémité, sur le col extérieur du tube et sur le col intérieur du tube de manière à soumettre l'intérieur du tube, le col extérieur et le col intérieur au rayonnement ultra-violet lors du passage du tube au niveau de la cavité (4); ledite tube comprenant :
- une deuxième extrémité (6) formant un fond du tube, et
- un corps (7) s'étendant entre la première extrémité et la deuxième extrémité;
le dispositif étant agencé que lors de la soumission du tube au rayonnement ultra-violet, la deuxième extrémité du tube ne se trouve pas dans ladite cavité (4).

## Patentansprüche

1. Verfahren zur Dekontamination eines Röhrchens (2), dessen erstes offenes Ende (3) einen Innenhals (3a) zur Innenseite des Röhrchens und einen Außenhals (3b) zur Außenseite des Röhrchens hin bildet, wobei das Verfahren umfasst:
- eine anfängliche Erhöhung der Röhrchentemperatur, dann
Aussetzen des Röhrchens gegenüber einer ultravioletten Strahlung, umfassend eine Emission der ultravioletten Strahlung durch eine Strahlung emittierende Einrichtung (5), und Aussetzen des Inneren des Röhrchens, des Außenhalses und des Innenhalses gegenüber der ultravioletten Strahlung, während das erste Ende (3) in das Innere eines Hohlraums (4) eingeführt wird, der so ausgestaltet ist, dass er die von der Emissionseinrichtung emittierte ultraviolette Strahlung reflektiert und diese ultraviolette Strahlung durch das erste Ende ins Innere des Röhrchens, zum Außenhals des Röhrchens und zum Innenhals des Röhrchens (2) lenkt; wobei das Röhrchen (2) umfasst:
- ein zweites Ende (6), das einen Boden des Röhrchens bildet, und
- einen Körper (7), der sich zwischen dem ersten Ende und dem zweiten Ende erstreckt;
so dass sich das zweite Ende des Röhrchens nicht im Hohlraum befindet, wenn das Röhrchen ultravioletter Strahlung ausgesetzt wird.

2. Verfahren nach Anspruch 1, **dadurch gekennzeichnet, dass,** wenn das Röhrchen ultravioletter Strahlung ausgesetzt wird, der Röhrchenkörper sich nicht im Hohlraum befindet.

3. Verfahren nach einem der vorhergehenden Ansprüche,
**dadurch gekennzeichnet, dass** alle Teile des Röhrchens oder alle Teile des Röhrchens mit Ausnahme des Halses eine Temperatur von über oder gleich 85 °C aufweisen, wenn das Röhrchen ultravioletter Strahlung ausgesetzt wird.

4. Verfahren nach einem der vorhergehenden Ansprüche,
**dadurch gekennzeichnet, dass,** wenn das Röhrchen ultravioletter Strahlung ausgesetzt wird, die Strahlungsemissionseinrichtung nicht in das Innere des Röhrchens eingeführt wird.

5. Verfahren nach Anspruch 4, **dadurch gekennzeichnet, dass** es nicht das Einführen einer anderen Strahlungsemissionseinrichtung in das Röhrchen umfasst.

6. Verfahren nach einem der vorhergehenden Ansprüche,
**dadurch gekennzeichnet, dass** die ultraviolette Strahlung mindestens eine UV-Wellenlänge zwischen 180 nm und 380 nm umfasst und Folgendes aufweist:
- eine Impulsdauer von weniger als 500 µs und/oder
- eine Lichtenergie pro Impuls von mindestens 70 Joule und/oder
- eine Impulsfrequenz von mindestens 0,5 Hz oder eine Impulserzeugung, die in Abhängigkeit von der Geschwindigkeit gesteuert wird, mit der die zu dekontaminierenden Röhrchen vor der Emissionseinrichtung (5) und/oder im Hohlraum (4) passieren.

7. Verfahren nach einem der vorstehenden Ansprüche,
**dadurch gekennzeichnet, dass** das Röhrchen ein Röhrchen aus Kunststoff ist.

8. Verfahren nach einem der vorhergehenden Ansprüche,
**dadurch gekennzeichnet, dass** es, nachdem das Röhrchen der ultravioletten Strahlung ausgesetzt wurde, mindestens einen der folgenden Schritte vorzugsweise in der folgenden Reihenfolge umfasst:
- Ändern der Form des Röhrchens und/oder
- Befüllen des Röhrchens und/oder
- Befestigen eines Verschlusses auf dem Röhrchen, der den Außenhals berührt.

9. Verfahren nach Anspruch 8, **dadurch gekennzeichnet, dass** es keine Erhöhung der Röhrchentemperatur umfasst:
- zwischen dem Aussetzen des Röhrchens gegenüber der ultravioletten Strahlung und der Änderung der Form des Röhrchens und/oder
- zwischen dem Aussetzen des Röhrchens gegenüber der ultravioletten Strahlung und dem Befüllen des Röhrchens und/oder
- zwischen dem Aussetzen des Röhrchens gegenüber der ultravioletten Strahlung und dem Befestigen des Verschlusses.

10. Verfahren nach Anspruch 8 oder 9, **dadurch gekennzeichnet, dass** es das Aussetzen des Verschlusses gegenüber einer ultravioletten Strahlung umfasst, bevor er auf dem Röhrchen befestigt wird.

11. Verfahren nach einem der vorstehenden Ansprüche,
**dadurch gekennzeichnet, dass** es keine chemische Dekontamination des Röhrchens umfasst.

12. Verfahren nach einem der vorhergehenden Ansprüche,
**dadurch gekennzeichnet, dass** die anfängliche Temperaturerhöhung dadurch erreicht wird, dass das Röhrchen einen Ofen durchläuft und/oder dass das Röhrchen einer Infrarotlampe ausgesetzt wird.

13. Verfahren nach einem der vorhergehenden Ansprüche,
**dadurch gekennzeichnet, dass** durch die anfängliche Temperaturerhöhung alle Teile des Röhrchens auf eine Temperatur von mindestens 90 °C gebracht werden.

14. Vorrichtung (1) zur Dekontamination eines Röhrchens (2), umfassend:
- eine Einrichtung (13) zum Bewegen eines Röhrchens, dessen erstes offenes Ende (3) einen Innenhals (3a) zur Innenseite des Röhrchens und einen Außenhals (3b) zur Außenseite des Röhrchens hin bildet, entlang eines Dekontaminationswegs in einer Beförderungsrichtung (14),
- eine Einrichtung zur anfänglichen Erwärmung (8), die so angeordnet ist, dass sie die Temperatur des Röhrchens auf dem Dekontaminationsweg erhöht,
- eine Einrichtung, um das Röhrchen einer ultravioletten Strahlung auszusetzen, umfassend eine Emissionseinrichtung (5), die dazu eingerichtet ist, die ultraviolette Strahlung zu emittieren, und einen Hohlraum (4), der sich auf dem Dekontaminationsweg stromabwärts der Heizeinrichtung relativ zur Beförderungsrichtung befindet, wobei die Beförderungseinrichtung (13) und der Hohlraum (4) so zusammen angeordnet sind, dass das erste Ende (3) während eines Durchlaufs des Röhrchens auf der Höhe des Hohlraums (4) in den Hohlraum (4) eingeführt wird,
wobei der Hohlraum (4) so ausgestaltet ist, dass er die von der Emissionseinrichtung emittierte ultraviolette Strahlung reflektiert und diese ultraviolette Strahlung durch das erste Ende in das Innere des Röhrchens, zum Außenhals des Röhrchens und zum Innenhals des Röhrchens lenkt, um das Innere des Röhrchens, den Außenhals und den Innenhals während des Durchlaufs des Röhrchens auf der Höhe des Hohlraums (4) ultravioletter Strahlung auszusetzen; wobei das Röhrchen umfasst:
- ein zweites Ende (6), das einen Boden des Röhrchens bildet, und
- einen Körper (7), der sich zwischen dem ersten Ende und dem zweiten Ende erstreckt;
wobei die Vorrichtung so angeordnet ist, dass sich das zweite Ende des Röhrchens nicht in dem Hohlraum (4) befindet, wenn das Röhrchen ultravioletter Strahlung ausgesetzt wird.

## Claims

1. Method for decontaminating a tube (2) a first open end (3) of which forms an inner neck (3a) towards the inside of the tube and an outer neck (3b) towards the outside of the tube, said method comprising:
- initially raising the temperature of the tube, then
- subjecting the tube to ultraviolet radiation, comprising emitting ultraviolet radiation by radiation emission means (5), and subjecting the inside of the tube, the outer neck and the inner neck to the ultraviolet radiation, when the first end (3) is introduced inside a cavity (4) arranged in order to reflect the ultraviolet radiation emitted by the emission means and to direct this ultraviolet radiation to the inside of the tube through the first end, onto the outer neck of the tube and onto the inner neck of the tube, said tube (2) comprising:
- - a second end (6) forming a tube bottom, and,
- - a body (7) extending between the first end and the second end so that, when the tube is subjected to ultraviolet radiation, the second end of the tube is not in the cavity.

2. Method according to claim 1, **characterized in that**, when the tube is subjected to the ultraviolet radiation, the body of the tube is not located in the cavity.

3. Method according to any one of the preceding claims, **characterized in that** all the parts of the tube or all the parts of the tube except for the neck are at a temperature above or equal to 85°C when the tube is subjected to the ultraviolet radiation.

4. Method according to any one of the preceding claims, **characterized in that**, when the tube is subjected to the ultraviolet radiation, the radiation emission means are not inserted inside the tube.

5. Method according to claim 4, **characterized in that** it does not comprise any insertion inside the tube of any other radiation emission means.

6. Method according to any one of the preceding claims, **characterized in that** the ultraviolet radiation comprises at least one UV wavelength comprised between 180 nm and 380 nm, and has:
- a pulse duration less than 500 µs, and/or
- a luminous energy per pulse of at least 70 joules, and/or
- a pulse frequency of at least 0.5 Hz or a pulse production controlled as a function of the rate of advance of the tubes to be decontaminated in front of the emission means (5) and/or in the cavity (4).

7. Method according to any one of the preceding claims, **characterized in that** the tube is a tube made from plastic material.

8. Method according to any one of the preceding claims, **characterized in that** it comprises at least one of the following steps after subjecting the tube to the ultraviolet radiation, preferably in the following order:
- modifying the tube shape, and/or
- filling the tube, and/or
- fixing a cap on the tube in contact with the outer neck.

9. Method according to claim 8, **characterized in that** it does not comprise any increase in the temperature of the tube.
- between subjecting the tube to the ultraviolet radiation and modifying the shape of the tube, and/or-
- between subjecting the tube to the ultraviolet radiation and filling the tube, and/or
- between subjecting the tube to the ultraviolet radiation and fixing the cap.

10. Method according to claim 8 or 9, **characterized in that** it comprises subjecting the cap to ultraviolet radiation before it is fixed on the tube.

11. Method according to any one of the preceding claims, **characterized in that** it does not comprise any chemical decontamination of the tube.

12. Method according to any one of the preceding claims, **characterized in that** the initial temperature increase is obtained by passing the tube through an oven and/or by subjecting the tube to an infrared lamp.

13. Method according to any one of the preceding claims, **characterized in that** the initial temperature increase raises all the parts of the tube to a temperature above or equal to 90°C.

14. Decontamination device (1) of a tube (2) comprising:
- means (13) for moving, along a decontamination path in a direction of travel (14), a tube a first open end (3) of which forms an inner neck (3a) towards the inside of the tube and an outer neck (3b) towards the outside of the tube,
- initial heating means (8), arranged in order to raise the temperature of the tube on the decontamination path,
- means for subjecting the tube to ultraviolet radiation, comprising emission means (5) arranged in order to emit the ultraviolet radiation, and a cavity (4) situated on the decontamination path downstream of the heating means with respect to the direction of travel, the movement means (13) and the cavity (4) being arranged together so that the first end (3) is introduced inside the cavity (4) when the tube arrives at the cavity (4).
- the cavity (4) being arranged in order to reflect the ultraviolet radiation emitted by the emission means and to direct this ultraviolet radiation to the inside of the tube through the first end, onto the outer neck of the tube and onto the inner neck of the tube so as to subject the inside of the tube, the outer neck and the inner neck to the ultraviolet radiation when the tube arrives at the cavity (4); said tube comprising :
comprising:
- - a second end (6) forming a tube bottom, and,
- a body (7) extending between the first end and the second end, said device being executed so that, when the tube is subjected to ultraviolet radiation, the second end of the tube is not in the cavity (4).
